# EUROPEAN PATENT APPLICATION

(11) **EP 2 386 346 A1**
(43) Date of publication of application: **16.11.2011**
(21) Application number: 10162501.0
(22) Date of filing: 11.05.2010
(51) Int. Cl.: B01D 53/14, C07C 273/04, C07C 273/16

(54) **Process for the reduction of ammonia emissions in a urea manufacturing process**

(71) Applicant: Stamicarbon B.V., 6135 KW Sittard (NL)
(72) Inventor: Meessen, Jo, 6135 KW, Sittard (NL); Odenbrand, Clas Ulf Ingemar, SE-226 55, Lund (SE); Brandin, Jan, 215 79, Malmö (SE)
(74) Representative: Swinkels, Bart Willem

(57) **Abstract**

The present invention relates to a process for removing ammonia from an effluent of an ammonia enriched gaseous stream formed in or downstream the finishing section of a urea manufacturing process, said ammonia enriched gaseous stream comprising 200 mg NH₃/Nm³ or less, wherein the ammonia enriched gaseous stream is contacted with an aqueous composition comprising phosphoric acid thereby producing an ammonia enriched liquid effluent, wherein a bleed of the ammonia enriched liquid effluent is subjected to a urea decomposition step. The present invention also relates to a process for producing an ammonium phosphate essentially free of contaminants, said process comprising the following steps: (a) contacting an ammonia enriched gaseous stream comprising 200 mg NH₃/Nm³ or less with an aqueous composition comprising phosphoric acid thereby producing an ammonia enriched liquid effluent; and (b) subjecting a bleed of the ammonia enriched liquid effluent to a urea decomposition step.

## Description

### Field of the invention

The present invention relates to a process for the reduction of ammonia emissions in a urea manufacturing process. The present invention also relates to a process for producing an ammonium phosphate essentially free of contaminants.

### Background of the invention

Urea manufacturing processes are well known in the art and exists already for a very long time. Kirk-Othmer, Encyclopedia of Chemical Technology, "Urea", pages 597 - 621, 4th Ed., Supplement Volume, 1998**,** and Ullmann's Encyclopdia of Industrial Chemistry, "Urea", J.H. Meessen and H. Petersen, 7th Ed., 2010**,** both incorporated by reference, disclose *inter alia* once-through processes, recycle processes and stripping processes. Basically, these processes comprise a synthesis section, a concentration section for evaporating water from the urea solution obtained from the synthesis section to produce a highly concentrated urea solution (often referred to as the "urea melt"), and a finishing section where the highly concentrated urea solution is prilled or granulated.

The manufacture of urea prills has *inter alia* the disadvantage that during the prilling process the highly concentrated urea solution must be cooled by air wherein a gaseous stream comprising very fine urea dust and ammonia is generated. Removal of these components from this gaseous stream is highly desirable for environmental reasons but that appears to be technically very complicated.

Granulation reduces very fine dust formation and is therefore more commonly applied in newer urea plants. The much coarser dust obtained in granulation processes is usually fed to a urea dust scrubbing section resulting into the formation of a recovered aqueous urea solution and a gaseous effluent which is released into the atmosphere. However, this gaseous effluent still comprises urea dust and ammonia in such amounts which today are still considered as unacceptable for environmental reasons.

GB 844.294, incorporated by reference, discloses a process wherein a gaseous effluent, said effluent comprising ammonia, carbon dioxide, water and inert gases, from the synthesis section of a urea production plant is worked up by selective absorption of ammonia which is then, after desorption, recycled to the manufacturing process. Small amounts of ammonia that remain in the gaseous effluent of the absorber are subsequently removed by washing with aqueous sulphuric acid. The ammonium sulphate formed is then added to the originally produced aqueous solution of urea, after which the mixture is concentrated and granulated. Hence, the process according to GB 844.294 is performed upstream of the finishing section of the urea production plant.

US 5.527.961, incorporated by reference, addresses the technical problem that gaseous effluents of urea manufacturing processes, in particular gaseous effluents from the granulation or prilling section, contain relatively high amounts of ammonia, i.e. about 80 - 200 and typically about 160 - 180 mg NH₃/Nm³ (or ppm), and that direct release of these gaseous effluents is environmentally unfriendly. It is therefore relevant and often even compulsory in view of legislation that the amounts of ammonia in such gaseous effluents are reduced to levels of about 10 mg NH₃/Nm³ or less. In the finishing step of the urea manufacturing process, molten urea is granulated or prilled wherein cooling air is used to remove ammonia from the molten urea. The effluent air stream is said to contain about 80 - 200 mg NH₃/Nm³ which, however, is said as not being easily removed from this effluent air stream by e.g. washing this stream with water. The solution provided by US 5.527.961 is a process wherein ammonia is already removed from molten urea by adding an acid, in particular sulfuric acid or phosphoric acid, in an amount of 0.25 - 0.45 wt.% on the molten urea, wherein the ammonia is converted into ammonium sulfate or ammonium phosphate. The ammonium sulfate or ammonium phosphate end up into the granulated or prilled urea which is, however, not very desirable since several urea end-uses require urea of high purity. Moreover, the use of corrosive acids requires more intensive plant control and maintenance and therefore higher operating costs. In addition, the process requires high investments in additional process equipment. On the other hand, by this process, the ammonia content of the gaseous effluent of a prilling tower could, according to the examples, be reduced to 4 - 5 mg NH₃/Nm³. Hence, the process according to US 5.527.961is not performed downstream of the finishing section of the urea production plant.

JP A 09-227493, incorporated by reference, discloses a process wherein the gaseous effluent air stream from the granulation or prilling section, said gaseous effluent air stream comprising 5000 - 15000 mg urea/Nm³ and 100 - 500 mg NH₃/Nm³, is fed to an exhaust air cleaning unit. The exhaust air cleaning unit comprises a packed bed over which an acidic aqueous urea solution having a pH of 3.5 to 5.5 is circulated, Upon contacting the gaseous effluent air stream with the acidic aqueous urea solution, an effluent air stream comprising less than 50 mg NH₃/Nm³ and an aqueous urea-ammonium salt solution is produced. The aqueous urea-ammonium salt solution is used as fertilizer. The pH of 3.5 to 5.5 is maintained by feeding an acid to the exhaust air cleaning unit, wherein the acid may be selected from the group consisting of nitric acid, hydrochloric acid, phosphoric acid and sulfuric acid.

JP A 2000-001466, incorporated by reference, discloses that it provides an improvement over the process disclosed in JP A 09-227493. According to JP 2000-001466, the gaseous effluent air stream from the granulation or prilling section is fed to a first washing tower comprising a packed bed wherein an aqueous urea solution circulates and wherein gaseous urea from gaseous effluent air stream is removed. The liquid effluent from the first washing tower is recycled to the concentration section. The gaseous effluent from the first washing tower is fed to a second washing tower comprising a packed bed wherein an acidic aqueous solution having a pH of 3.5 to 7.0 circulates and wherein ammonia is removed as ammonium salt. The ammonium salt is recycled to the granulation section and ends up in the urea granules. Hence, the urea produced according to the process of JP 2000-001466 can essentially only be used as fertilizer since it contains significant amounts of ammonium salts. The acid for maintaining the pH at 3.5 to 7.0 may be selected from the group consisting of nitric acid, hydrochloric acid, phosphoric acid and sulfuric acid.

EP 440.932, incorporated by reference, discloses a process wherein ammonia is removed from an effluent gas comprising ammonia and optionally dust, said effluent gas originating from a fertilizer production process, in an absorption column through which an acidic ammonium nitrate solution is circulated. The process encompasses a bleed wherein ammonium nitrate is fed to the fertilizer production process.

EP 514.902, incorporated by reference, discloses a process wherein the gaseous effluent air stream from the granulation or prilling section is subjected to a scrubbing step in order to remove ammonia. In this scrubbing step, the gaseous effluent air stream is contacted with an aqueous solution of urea and an inorganic acid such as sulfuric acid, phosphoric acid, nitric acid or a mixture of these acids. The pH of the aqueous solution is 5.0 to 7.0, preferably 5.5 to 6.5, in order to limit undesired reactions such as hydrolysis of urea.

US 2007/0039469, incorporated by reference, discloses a process for removing ammonia from gaseous effluents of in particular the finishing section of a urea manufacturing process, wherein the process comprises feeding the waste gas to a first washer comprising a fine-washing area and a main washing area. According to a preferred embodiment, the gaseous effluent first passes a pre-purification stage and subsequently enters the main washing area and the fine-washing area, respectively, of the first washer. According to another preferred embodiment, an acid, e.g. sulfuric acid or nitric acid, is introduced in the fine-washing area. Purified gas exits the head of the first washer. However, process conditions and emission reductions achievable by the described process are not disclosed.

WO 2009/138178, incorporated by reference, discloses a process for reducing aerosols emissions from a urea granulation plant, wherein the gaseous effluent comprising urea dust, ammonia and ammonium isocyanate is fed to a dust scrubbing stage and subsequently to an aerosol separation stage. The bleed of the aerosol separation stage which comprises ammonium isocyanate and fine urea dust is fed via a heat exchanger to a stripping column (which also functions as an ammonium isocyanate isomerisation unit), where it is separated into a gaseous top stream comprising ammonia, carbon dioxide and water and an aqueous bottom stream comprising urea. The gaseous effluent of the aerosol separation stage passes an acid scrubber and is subsequently released into the atmosphere. The aqueous bottom stream comprising urea is concentrated and recycled to the finishing section or the dust scrubbing section or it is used for other purposes. The nature of the acid used is not disclosed. Nor are process conditions or emission reductions achievable by the described process disclosed.

Processes for converting urea into ammonia and carbon dioxide are also known in the art. For example, US 3.826.815 and US 3.922.222, both incorporated by reference, disclose a process for the manufacture of melamine, which process includes a step wherein urea is hydrolyzed in the presence of water vapor at a pressure of at least about 20 p.s.i.g. (about 0.14 MPa) and 8.4 p.s.i.a. (about 0.16 MPa), respectively.

NL 8105027, incorporated by reference, discloses a catalytic process wherein a gaseous stream of urea and of other compounds containing nitrogen, e.g. melamine, is contacted with water vapor at a temperature of 100°C to 500°C. The process is suitable for treating the gaseous effluent air stream obtained from the concentration section or the gaseous effluent air stream from the granulation or prilling section.

US 4.087.513, incorporated by reference, discloses a process for hydrolyzing urea present in waste streams from the concentration section. The waste stream contains about 0.01 to about 20 wt.% of urea. The urea present in the waste stream is hydrolyzed in a CO₂ recovery system which is part of an ammonia production process. According to US 4.168.299 and US 4.220.635, incorporated by reference, vanadium pentoxide and other vanadium compounds may be used as a catalyst.

US 4.341.640, incorporated by reference, discloses a process for hydrolyzing urea wherein a urea containing waste stream is fed to a column which is maintained at a temperature of 120°C to 250°C and a pressure of 30 to 300 p.s.i.g. (about 0.2 to 2 MPa).

US 4.410.503, incorporated by reference, discloses a process for treating the process condensate comprising urea which is formed in the synthesis section with steam at elevated pressure and temperature.

EP 53.410, incorporated by reference, discloses a process for hydrolyzing urea in a column by countercurrent contact with high pressure steam.

EP 417.829, incorporated by reference, discloses a process for purifying waste water streams from a urea plant which includes a step for hydrolyzing urea, said step being performed at 20 to 40 bar (2 to 4 MPa) and a temperature of 200°C to 240°C.

US 5.252.308 discloses a process for manufacturing ammonia from urea, wherein urea is treated with sulfuric acid or phosphoric acid at a temperature of at least 45°C to form ammonium sulfate or ammonium phosphate and carbon dioxide, wherein the ammonium sulfate or ammonium phosphate is converted into ammonia at a temperature of at least 130°C.

In Selective Catalytic Reduction (SCR) or Selective Non-Catalytic Reduction (SNCR) methods for removal of nitrogen oxides, gaseous ammonia is used which is generated from aqueous solutions of urea at high temperatures.

The processes known from the prior art for treating the gaseous effluent air stream from the granulation or prilling section art have certain drawbacks. For example, in pre-finishing section processes, compounds are formed which end up in the final product, i.e. the prilled or granulated urea and therefore also enter the environment. Post-finishing section processes do not account for urea removal but only for ammonia removal. Although processes for hydrolysying urea are known, these processes are conducted at high temperature and/or high pressure and optionally need the presence of a catalyst.

### Summary of the invention

The present invention provides a process for efficiently removing ammonia as well as urea from the gaseous effluent air stream from the granulation or prilling section of a urea manufacturing process, wherein high temperatures and/or high pressures are not necessary. The process provides urea that is not contaminated with ammonium salts. Instead, a pure ammonium salt product is produced.

Accordingly, the present invention relates to a process for removing ammonia from an effluent of an ammonia enriched gaseous stream formed in or downstream the finishing section of a urea manufacturing process, said ammonia enriched gaseous stream comprising 200 mg NH₃/Nm³ or less, wherein the ammonia enriched gaseous stream is contacted with an aqueous composition comprising phosphoric acid thereby producing an ammonia depleted liquid effluent, wherein a bleed of the ammonia depleted liquid effluent is subjected to a urea decomposition step.

The present invention further relates to a process for producing an ammonium phosphate essentially free of contaminants, in particular urea.

The process according to the present invention has several distinct advantages over the processes of the prior art. First, the urea decomposition step of the process can be run at low pressure and low temperature which is beneficial in terms of investment, maintenance and operation cost and energy consumption. Secondly, the process according to the present invention provides ammonium phosphate which is not contaminated with urea. Additionally, the process enables the manufacture of urea without contamination with ammonium phosphate.

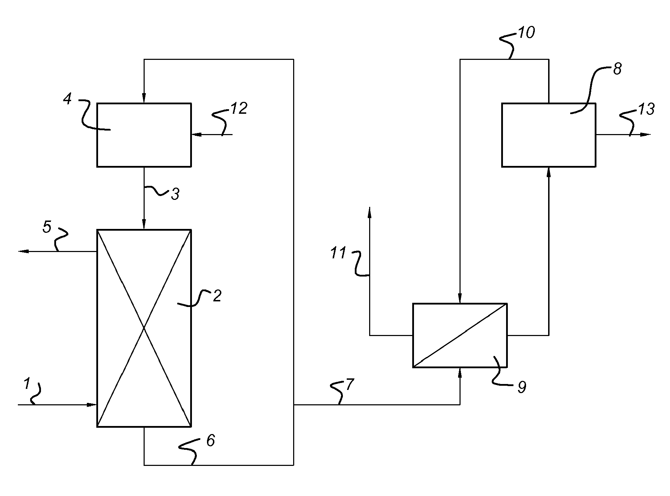

### Brief description of Figure 1

The figure shows a block diagram of the process according to a preferred embodiment of the present invention.

### Detailed description of the invention

The verb "to comprise" as is used in this description and in the claims and its conjugations is used in its non-limiting sense to mean that items following the word are included, but items not specifically mentioned are not excluded. In addition, reference to an element by the indefinite article "a" or "an" does not exclude the possibility that more than one of the element is present, unless the context clearly requires that there is one and only one of the elements. The indefinite article "a" or "an" thus usually means "at least one".

The term "an ammonia enriched gaseous stream formed in or downstream the finishing section of a urea manufacturing process" is to be understood as an effluent of the finishing section, i.e. the section where the highly concentrated urea solution is prilled or granulated, or an effluent formed further downstream of said finishing section, e.g. in a urea dust scrubbing section.

According to the present invention, the ammonia enriched gaseous stream comprises 200 mg NH₃/Nm³ or less, preferably about 1 to about 200 mg NH₃/Nm³, more preferably 180 mg NH₃/Nm³ or less, even more preferably 170 mg NH₃/Nm³ or less, yet even more preferably 160 mg NH₃/Nm³ or less, and in particular 150 mg NH₃/Nm³ or less. The ammonia enriched gaseous stream preferably comprises at least 10 mg NH₃/Nm³, more preferably at least 20 mg NH₃/Nm³, even more preferably at least 30 mg NH₃/Nm³, yet even more preferably 40 mg NH₃/Nm³, and in particular at least 50 mg NH₃/Nm³. However, as will be apparent to those skilled in the art, the ammonia content of the ammonia enriched gaseous stream is as low as possible.

Additionally, the ammonia enriched gaseous stream comprises 100 mg urea/Nm³ or less, preferably 75 mg urea/Nm³ or less, and in particular 50 mg urea/Nm³ or less. The minimum amount is preferably 1 mg urea/Nm³, preferably 5 mg urea/Nm³, and in particular 10 mg urea/Nm³. However, as will be apparent to those skilled in the art, the urea content of the ammonia enriched gaseous stream is as low as possible.

The air humidity of the ammonia enriched gaseous stream is preferably about 0.01 to about 0.5 kg/kg, more preferably about 0.02 to 0.2 kg/kg and most preferably about 0.03 to about 0.1 kg/kg, based on the total weight of the ammonia enriched gaseous stream.

The flow of the ammonia enriched gaseous stream entering the process according to the present invention is preferably expressed as the amount of the ammonia enriched gaseous stream in ratio to the amount of urea processed in the finishing section. This amount is preferably about 5 to about 30 kg, more preferably about 8 to about 15 kg, of ammonia enriched gaseous stream per kg of urea that is processed in the finishing section of the urea plant.

According to an embodiment of the present invention, the flow of the ammonia enriched gaseous stream entering the process is about 100.000 Nm³/h to about 2.000.000 Nm³/h.

According to an embodiment of the present invention, the ammonia enriched gaseous stream is the gaseous effluent of the finishing section, where it is preferred that the finishing section is the granulation of a urea manufacturing process. According to another embodiment of the present invention, the ammonia enriched gaseous effluent of the finishing section may first be fed to another (pre-)purification section, preferably a urea dust scrubbing section, such as is for example disclosed in WO 02/46145, incorporated by reference, wherein the gaseous effluent of the (pre-)purification section, preferably the urea dust scrubbing section, is the ammonia enriched gaseous stream which is contacted with the aqueous composition comprising phosphoric acid.

If the process of the present invention encompasses a urea dust scrubbing section, the liquid scrubber effluent is preferably recycled to the synthesis section of a urea manufacturing process.

According to a preferred embodiment of the present invention, the ammonia enriched gaseous stream is fed to an absorber. This ammonia enriched gaseous stream may be either the gaseous effluent of the finishing section or the gaseous effluent of the (pre-)purification section, preferably the urea dust scrubbing section. Most preferably, the ammonia enriched gaseous stream is the gaseous effluent of the urea dust scrubbing section.

Preferably, (i) the aqueous composition comprising phosphoric acid and (ii) the ammonia enriched gaseous stream are contacted countercurrently, wherein an ammonia depleted gaseous effluent and an ammonia enriched liquid effluent are produced. The conversion of ammonia per pass is preferably greater than 95%.

According to the present invention, the aqueous composition comprising phosphoric acid has preferably a pH of less than 5, more preferably less than 4, yet even more preferably less than 3 and in particular less than 2. The aqueous composition comprising phosphoric acid has preferably a pH higher than 1, more preferably higher than 1.1.

The aqueous composition comprising phosphoric acid and the ammonia enriched gaseous stream are preferably contacted at a temperature of about 20° to about 80°C, preferably about 20° to about 70°C, and in particular about 20° to about 60°C.

According to the present invention, the ammonia depleted gaseous effluent that is produced in the process according to the present invention comprises less than 10 mg NH₃/Nm³. This ammonia depleted gaseous effluent can be released to the atmosphere since the ammonia content is considered to be within environmentally acceptable limits.

The ammonia enriched liquid effluent of the contacting step involving the aqueous composition comprising phosphoric acid is preferably recycled, preferably to the absorber. It is also preferred that a bleed of the ammonia enriched liquid effluent is subjected to a urea decomposition step.

In the urea decomposition step, urea is decomposed into carbon dioxide and ammonia. Since the ammonia enriched liquid effluent is acidic and has preferably a pH in the range described for the aqueous composition comprising phosphoric acid, any ammonia formed will be converted into ammonium phosphate. It is preferred that the urea decomposition step is conducted at a temperature of about 20° to below about 150°C, more preferably at about 50° to about 150°C, yet more preferably at about 60°C to about 150°C, even yet more preferably at about 60° to about 130°C, even yet more preferably at about 60°C to about 95°C, and most preferably about 70° to about 90°C. In addition, it is preferred that the urea decomposition step is run at about atmospheric pressure.

Optionally, the liquid effluent of the urea decomposition step or a part thereof which comprises very pure ammonium phosphate, is recycled, e.g. to the absorber where it can be used to adjust the pH of the aqueous composition comprising phosphoric acid. Alternatively, this liquid effluent or a part thereof is used in the manufacture of a fertilizer. It is, however, preferred that this liquid effluent is used in the manufacture of a fertilizer.

It is preferred that the effluent of the urea decomposition step is cooled to a temperature of about 20° to about 40°C, preferably to ambient temperature, prior to any further processing. In this embodiment, it is advantageous that the effluent of the urea decomposition step exchanges heat with the ammonia enriched liquid effluent that is subjected to the urea decomposition step.

The present invention also relates to a process for producing an ammonium phosphate essentially free of contaminants, said process comprising the following steps:
(a) contacting an ammonia enriched gaseous stream comprising 200 mg NH₃/Nm³ or less with an aqueous composition comprising phosphoric acid thereby producing an ammonia enriched liquid effluent; and
(b) subj ecting a bleed of the ammonia enriched liquid effluent to a urea decomposition step.

The ammonia enriched gaseous stream, the aqueous composition comprising phosphoric acid, the ammonia enriched liquid effluent, the bleed of the ammonia enriched liquid effluent and the urea decomposition step are described above.

The figure shows a block diagram of the process according to a preferred embodiment of the present invention. Stream 1 represents the incoming ammonia enriched gaseous stream which may originate from the finishing section (not shown) or from the (pre-)purification section, preferably a urea dust scrubbing section (not shown), and is fed to absorber 2. Stream 3 represents the aqueous composition comprising phosphoric acid and ammonium dihydrogen phosphate which is for example provided by a dosing tank 4. The phosphoric acid is fed to dosing tank 4 via line 12. Stream 3 and stream 1 are contacted countercurrently in absorber 2. The ammonia depleted gaseous effluent 5 leaves the absorber at the top. It is preferred that the absorber is of such design that it can cope with the whole flow of the ammonia enriched gaseous stream and that it, if necessary, comprises a multitude of parallel absorbers. The ammonia enriched liquid effluent 6 is partly recycled to the dosing tank 4 and partly fed as a bleed 7 to the urea decomposition reactor 8 wherein it passes a heat exchanger 9 which operates in conjunction with a heater (not shown). The bleed 7 is heated by the heater and by exchanged heat with the effluent 10 from the urea decomposition step which needs to be cooled prior to further processing to the desired temperature. The carbon dioxide formed in the urea decomposition step leaves the urea decomposition reactor 8 via line 13. The effluent 10 from the urea decomposition step leaves the heat exchanger 9 as stream 11.

### Example

The process according to the present invention is exemplified by the process as shown in Figure 1. The numbers given are related to one hour of operation. The mass balances are based on absorption in a mixture of 0.5 M NH₄H₂PO₄ a 0.5 M H₃PO₄ in water. An ammonia enriched gaseous stream of 750.000 Nm³ comprising 41.1 Nm³ of H₂O (g), 6.618 kmol NH₃ (g) and 0.265 kmol urea (s) was fed via line 1 to an absorber 2 where it was contacted with 0.5 M phosphoric acid (H₃PO₄) and 0.5 M ammonium dihydrogenphosphate (NH₄H₂PO₄) which are fed from a storage tank 4 via line 3 to the absorber 2. To storage tank 4,6.18 kmol H₃PO₄ and 12.5 m³ H₂O are fed via line 12. An ammonia depleted gaseous stream of 750.000 Nm³ comprising 0.443 kmol NH₃ (g) (about 6% of the original amount that entered the absorber) left the absorber via line 5. The ammonia enriched liquid effluent having a temperature of about 25°C was discharged from the absorber via line 6 with a rate of 12.5 m³/h. The amount of carbon dioxide produced in the urea decomposition reactor 8 was 0.265 kmol which leaves the decomposition reactor 8 via line 13.. Effluent 10 was discharged from the urea decomposition reactor at a rate of 12.5 m³/h and was cooled to about 25°C.

## Claims

1. A process for removing ammonia from an effluent of an ammonia enriched gaseous stream formed in or downstream the finishing section of a urea manufacturing process, said ammonia enriched gaseous stream comprising 200 mg NH₃/Nm³ or less, wherein the ammonia enriched gaseous stream is contacted with an aqueous composition comprising phosphoric acid thereby producing an ammonia enriched liquid effluent, wherein a bleed of the ammonia enriched liquid effluent is subjected to a urea decomposition step.

2. The process according to Claim 1, wherein the gaseous stream is the gaseous effluent from the finishing section of the urea manufacturing process.

3. The process according to Claim 1 or Claim 2, wherein the finishing section is a granulation section.

4. The process according to any one of Claims 1 - 3, wherein the ammonia enriched gaseous stream is fed to an absorber.

5. The process according to any one of Claims 1 - 3, wherein the ammonia enriched gaseous stream passes a urea dust scrubbing section.

6. The process according to Claim 5, wherein the liquid scrubber effluent is recycled to the synthesis section of a urea manufacturing process.

7. The process according to Claim 5, wherein the gaseous scrubber effluent is fed to an absorber.

8. The process according to any one of Claims 4 - 7, wherein (i) the aqueous composition comprising phosphoric acid and (ii) the ammonia enriched gaseous stream are contacted countercurrently, wherein an ammonia depleted gaseous effluent and an ammonia enriched liquid effluent are produced.

9. The process according to Claim 8, wherein (i) the aqueous composition comprising phosphoric acid and (ii) the ammonia enriched gaseous stream are contacted at a temperature of about 20° to about 80°C.

10. The process according to Claim 8 or Claim 9, wherein the ammonia depleted gaseous effluent comprises less than 10 mg NH₃/Nm³.

11. The process according to any one of Claims 8 - 10, wherein the ammonia enriched liquid effluent is recycled.

12. The process according to any one of Claims 8 - 11, wherein the urea decomposition step is conducted at a temperature of about 60° to about 150°C.

13. The process according to any one of Claims 1 - 12, wherein the liquid effluent of the urea decomposition step is recycled.

14. The process according to Claim 1 - 13, wherein the effluent of the urea decomposition step is cooled to a temperature of about 20° to about 40°C.

15. A process for producing an ammonium phosphate essentially free of contaminants, said process comprising the following steps:
(a) contacting an ammonia enriched gaseous stream comprising 200 mg NH₃/Nm³ or less with an aqueous composition comprising phosphoric acid thereby producing an ammonia enriched liquid effluent; and
(b) subj ecting a bleed of the ammonia enriched liquid effluent to a urea decomposition step.
